# EUROPÄISCHE PATENTSCHRIFT

(11) **EP 2 570 807 B1**
(45) Veröffentlichungstag und Bekanntmachung des Hinweises auf die Patenterteilung: **28.10.2015**
(21) Anmeldenummer: 11181594.0
(22) Anmeldetag: 16.09.2011
(51) Int. Cl.: G01N 33/00, G08B 29/22

(54) **Prüfgerät zur Feldkalibrierung eines Gasmelders**
Test device for field calibration of a gas sensor
Appareil de contrôle pour le calibrage de champ d'un détecteur de gaz

(43) Veröffentlichungstag der Anmeldung: 20.03.2013
(73) Patentinhaber: Siemens Schweiz AG, 8047 Zürich (CH)
(72) Erfinder: Duric, Aleksandar, 6300 Zug (CH); Ebner, Harald, 6340 Baar (CH); Forster, Martin, 8645 Jona (CH)
(74) Vertreter: Maier, Daniel Oliver

(56) Entgegenhaltungen:
- WO-A2-01/65211
- DE-A1-102008 015 145
- US-A- 5 659 125
- US-A1- 2006 156 789
- US-A1- 2010 326 165

## Beschreibung

Die Erfindung betrifft die Feldkalibrierung von Gasmeldern. Hierbei wird die Konzentration eines Referenzgases bestimmt. Die Referenzgaskonzentration wird dabei entweder berechnet bzw. eine bekannte Menge Referenzgas wird in die Prüfkammer eines Prüfgerätes eingespeist und anschließend gemessen oder aber es wird eine unbekannte Konzentration des Referenzgases mit Hilfe eines Referenzgassensors gemessen. Die vorliegende Erfindung betrifft den zuletzt beschriebenen Fall und insbesondere die Frage, wie sichergestellt werden kann, dass der Referenzsensor des zur Feldkalibrierung verwendeten Prüfgerätes, der selbst Alterung und Umgebungseinflüssen ausgesetzt ist, korrekte Messwerte ausgibt.

Es ist bekannt, die Prüfgeräte in regelmäßigen Abständen neu zu kalibrieren. Dies geschieht im Werk oder in einem speziellen Prüflabor. Dieses Vorgehen ist aufwendig und kostenintensiv und für die im Feld breitgestreuten Prüfgeräte unpraktisch.

Aus der deutschen Offenlegungsschrift DE 10 2008 015 145 A1 ist ein Verfahren zur Nachkalibrierung von Sensoren und zur Kalibrierung weiterer Sensoren offenbart. Bei dem Verfahren wird ein erster Sensor durch Messen des Zielstoffes bei unterschiedlichen, dosierten Konzentrationen aus dem gesamten Kalibrierfeld kalibriert und dann die zugehörigen Rohdaten ermittelt. Danach werden für einen zweiten und für weitere Sensoren bei einer Referenzkonzentration aus dem Kalibrierfeld die entsprechenden Rohdaten bestimmt.

Der dortigen Erfindung liegt die Erkenntnis zugrunde, dass bei der industriellen Fertigung im Batchbetrieb nicht nur einzelne Sensorelemente sondern eine Vielzahl von baugleichen Sensorelementen zu kalibrieren sind. Mit baugleichen Sensorelementen werden aufgrund begrenzter Reproduzierbarkeit bei der Herstellung unterschiedliche Rohdaten erhalten. Allerdings weisen diese eine Ähnlichkeit auf. So unterscheiden sich z.B. die Leitwertzeitprofile im Wesentlichen in ihrem absoluten Niveau und/oder in einer vertikalen Streckung.

Es wird dann eine Funktion f* ausgewählt, die die Rohdaten vom ersten Sensor bei der Referenzkonzentration am besten auf die Rohdaten vom zweiten Sensor abbildet. In diesem Fall müssen nur für ein Sensorelement die Rohdaten des gesamten Kalibrierfeldes aufgenommen werden. Für alle anderen Sensorelemente müssen diese nur noch für eine Konzentration anstelle von 5 Konzentrationen aufgenommen werden, so dass ein Reduktionsfaktor 5 für alle anderen Sensorelemente aus der Batchfabrikation gilt.

Eine Aufgabe der vorliegenden Erfindung ist es, die Feldkalibrierung von Gasmeldern zu vereinfachen. Diese Aufgabe wird durch zwei Prüfgeräte nach den Ansprüchen 1 und 5 gelöst. Vorteilhafte Ausführungen der Erfindung sind in den Unteransprüchen angegeben.

Eine Kernidee der Erfindung ist es, wenigstens einen fabrikneuen Gasmelder als Normal zu verwenden, also als Vergleichsgerät zur Kalibrierung des Gasmelders im Feld. Dadurch wird sichergestellt, dass die Empfindlichkeit der geprüften Gasmelder im Feld innerhalb eines vorgegebenen Bereiches liegt.

Unter dem Begriff "Gasmelder" werden dabei Gasmelder und Gas-Rauchmelder verstanden, wobei unter einem Gas-Rauchmelder ein Rauchmelder, der auch noch ein Gas misst, und unter einem Gasmelder ein Gasmelder, der irgendein Gas misst, verstanden wird. Insbesondere betrifft die Erfindung die Feldkalibrierung von CO-Gas-Rauchmeldern, also Rauchmeldern, die Rauch und Kohlenmonoxid messen, bzw. CO-Gasmeldern, also Gasmeldern, die Kohlenmonoxid messen. Die Erfindung ist jedoch nicht auf Kohlenmonoxid-Gas messende Melder beschränkt. Auch Melder, die ein anderes Gas oder mehrere Gase messen, sind von dem nachfolgend allgemein verwendeten Begriff "Gasmelder" mit umfasst, ebenso Melder, die zusätzlich eine oder mehrere weitere Sensoranordnungen aufweisen, z. B. zur Temperaturmessung.

Unter "fabrikneu" wird dabei verstanden, dass es sich um einen neu produzierten, bisher nicht verwendeten und daher auch nur unwesentlich gealterten Gasmelder ("out of the box") handelt, der eine für Kalibrierzwecke ausreichende Qualität aufweist. Mit anderen Worten bedeutet "fabrikneu", dass sich die Empfindlichkeit des Gasmelders seit seiner letzten Kalibrierung, die bei seiner Herstellung im Werk stattfand, nur unwesentlich verändert hat. Unter "fabrikneu" wird auch ein neu kalibrierter oder überarbeiteter Gasmelder verstanden, der hinsichtlich seiner Eignung als Normal einem neu hergestellten Gasmelder entspricht.

Das kostenintensive und unpraktische Kalibrieren der Prüfgeräte im Werk oder in einem speziellen Prüflabor wird erfindungsgemäß dadurch vermieden, dass entweder die Feldkalibrierung mit wenigstens einem Prüfgerät durchgeführt wird, das sich eines fabrikneuen Gasmelders als Referenz bedient, oder aber, dass das Prüfgerät vor der Feldkalibrierung mit Hilfe wenigstens eines fabrikneuen Gasmelders kalibriert wird.

Anders ausgedrückt wird der wenigstens eine zweite, fabrikneue Gasmelder, dessen Empfindlichkeit bekannt ist, entweder als Gebrauchsnormal, nämlich als Referenzgassensor des Prüfgerätes, oder aber als Bezugsnormal für ein Gebrauchsnormal, also als Referenz für den Referenzgassensor des Prüfgerätes verwendet.

Anwendung der Erfindung beinhaltet mit anderen Worten die Verwendung wenigstens eines zweiten, fabrikneuen Gasmelders als Normal, und zwar entweder bei der Kalibrierung eines ersten Gasmelders oder aber bei der Kalibrierung eines Prüfgerätes, welches zur Kalibrierung eines ersten Gasmelders verwendet wird. Dabei sind zwei Ausführungsvarianten möglich, die nachfolgend näher beschrieben werden. In der ersten Verwendungsvariante weist das Verfahren zur Kalibrierung des Gasmelders folgende Schritte auf: Bestimmung der Konzentration eines Referenzgases durch Messung mit einem Gassensor des zu überprüfenden Gasmelders, Bestimmung der Konzentration des Referenzgases durch Messung mit dem Gassensor wenigstens eines als Gebrauchsnormal dienenden zweiten, fabrikneuen Gasmelders, Ermittlung der Abweichung der bei der Messung mit dem Gasmelder bestimmten Konzentration von der bei der Messung mit dem wenigstens einen zweiten Gasmelder bestimmten Konzentration sowie Verarbeitung der ermittelten Abweichung derart, dass diese bei einer anschließenden Benutzung des Gasmelders zur Korrektur einer Messung der Gaskonzentration berücksichtigt wird. Anders ausgedrückt erfolgt bei der Kalibrierung ein Messvorgang zur Feststellung und Dokumentation der Abweichung der Messwerte des zu kalibrierenden Gasmelders von den Messwerten des als Gebrauchsnormal dienenden wenigstens einen zweiten Gasmelders. Außerdem wird die ermittelte Abweichung bei der anschließenden Verwendung des Gasmelders zur Korrektur der gemessenen Werte berücksichtigt. Hierzu wird die ermittelte Abweichung verarbeitet, wobei hierunter zumindest das Ablegen in einem in dem Gasmelder enthaltenen Speicher und/oder das Übertragen an einen Empfänger, beispielsweise eine entsprechende Datenbank oder eine Brandmeldezentrale verstanden wird.

Bei diesem Verfahren erfolgt eine Kalibrierung zwar nicht mit Hilfe eines Werknormals, mit dem bei der Herstellung der Brandmelder die Gassensoren kalibriert werden. Die Kalibrierung erfolgt jedoch unter Verwendung wenigstens eines Referenzgassensors, dessen Genauigkeit sehr nahe an dem Werknormal liegt. Da die fabrikneuen, kalibrierten zweiten Brandmelder vom Prüfpersonal im Feld nach und nach verbaut werden und somit für die Feldkalibrierung ständig neue zweite Gasmelder als Gebrauchsnormale herangezogen werden, ist deren Empfindlichkeitsdegradation vernachlässigbar.

Das zur Durchführung dieses Verfahrens angepasste Prüfgerät zeichnet sich dadurch aus, dass es für eine gleichzeitige Messung der Gaskonzentration durch zwei oder mehr Gasmelder ausgebildet ist. Das Prüfgerät benötigt keinen eigenen Referenzgassensor. Statt dessen ist die Kalibrierelektronik des Prüfgerätes derart ausgebildet, dass sie mit dem wenigstens einen zweiten Gasmelder kommuniziert und nach einer gleichzeitigen Referenzmessung der Gaskonzentration eines Referenzgases durch den ersten und den wenigstens einen zweiten Gasmelder den zu kalibrierenden Gasmelder mit dem wenigstens einen zweiten Gasmelder als Gebrauchsnormal kalibriert. Der für zwei oder mehr Gasmelder ausgeführte Adapter ist derart ausgebildet, dass er eine einheitliche Gaskonzentration an allen angeschlossenen Gasmeldern sicherstellt.

Hierzu ist entweder ein gemeinsames Prüfvolumen für die Gasmelder vorgesehen oder mehrere voneinander getrennte Prüfvolumen, die den jeweiligen Gasmeldern zugeordnet sind. Im einfachsten Fall wird der zu überprüfende, erste Gasmelder abgenommen und gemeinsam mit dem wenigstens einen zweiten Gasmelder in ein durch einen Deckel verschließbaren, gemeinsamen Prüfraum des Prüfgerätes gelegt. In einer weiteren Ausführungsform der Erfindung weist das Prüfgerät einen Verbindungsadapter für wenigstens zwei Gasmelder auf. Ist das Prüfgerät entsprechend ausgebildet, verfügt der Adapter beispielsweise über entsprechende Dichtungselemente, wie Dichtlippen oder dergleichen, so ist es auch möglich, den ersten Gasmelder im montierten Zustand zu überprüfen.

Der Kalibriervorgang erfolgt entweder derart, dass der zu prüfende Gasmelder von seiner Betriebsposition abgenommen und an das Prüfgerät angeschlossen wird. Alternativ dazu ist es in einer Ausführungsform der Erfindung vorgesehen, den zu prüfenden Gasmelder in seiner Betriebsposition zu belassen. Dann ist das Prüfgerät derart ausgebildet, dass sichergestellt ist, dass der wenigstens eine zweite Gasmelder ebenso wie der zu prüfende Gasmelder gasdicht mit dem Prüfvolumen befestigt ist. Insbesondere dann, wenn der zu prüfende Gasmelder in seiner Betriebsposition kalibriert werden soll, muss darüber hinaus sichergestellt sein, dass beide Gasmelder auf derjenigen Seite, die der Referenzgaszuleitung abgewandet ist, gleich gasdicht sind, um ein identisches Ausströmverhalten des Referenzgases bei beiden Gasmeldern zu gewährleisten.

In der zweiten Verwendungsvariante wird das eigentliche Kalibrieren des Gasmelders im Prinzip ebenso ausgeführt, wie im Zusammenhang mit der ersten Variante beschrieben, jedoch mit dem Unterschied, dass als Gebrauchsnormal nicht der wenigstens eine zweite Gasmelder, sondern das Prüfgerät mit seinem eigenen Referenzgassensor verwendet wird. Das Prüfgerät wird jedoch zuvor mit dem zweiten Gasmelder als Bezugsnormal kalibriert. Es erfolgt mit anderen Worten zunächst eine Kalibrierung des Prüfgerätes mit eingebautem Referenzgassensor unter Verwendung des wenigstens einen zweiten Gasmelders als Bezugsnormal. Das neu kalibrierte Prüfgerät dient anschließend als Gebrauchsnormal zur Kalibrierung des ersten Gasmelders im Feld. Die Kalibrierung des Prüfgerätes kann vor jeder Feldkalibrierung eines Gasmelders erfolgen. Die Referenzgassensoren in dem Prüfgerät können beliebig stark altern. Da sie vor der Feldkalibrierung stets neu kalibriert werden, ist dies für die Qualität der Feldkalibrierung nicht von Belang, sofern sie die für den Anwendungszweck erforderliche und üblicherweise durch Normen vorgegebene Mindestempfindlichkeit und Dynamik aufweisen. Ist dies nicht mehr der Fall, werden sie durch neue Referenzgassensoren ersetzt.

Diese Ausführungsform der Erfindung, bei der der wenigstens eine zweite Gasmelder als Bezugsnormal für ein Gebrauchsnormal verwendet wird, zeichnet sich mit anderen Worten dadurch aus, dass wenigstens ein Referenzgassensor eines Prüfgerätes durch wenigstens einen fabrikneuen zweiten Gasmelder kalibriert wird und dass der zu kalibrierende erste Gasmelder durch den wenigstens einen Referenzgassensor kalibriert wird. Die beiden Kalibrierungen werden nacheinander ausgeführt, wobei die Reihenfolge der Messungen der Referenzgaskonzentration, welche den Kalibrierungen zugrunde liegen, keine Rolle spielt.

Das zur Durchführung dieses Verfahrens angepasste Prüfgerät zeichnet sich dadurch aus, dass dessen Adapter lediglich zur Herstellung einer Verbindung mit einem einzelnen Gasmelder ausgebildet sein muss, was den konstruktiven Aufbau des Prüfgerätes gegenüber der ersten Ausführungsform stark vereinfacht. Allerdings muss das jetzt zur Anwendung kommende Prüfgerät über wenigstens einen Referenzgassensor verfügen. Die mit dem Referenzgassensor verbundene Kalibrierelektronik ist zur Kommunikation mit dem wenigstens einen zweiten Gasmelder und zur Kalibrierung des ersten Gasmelders mit dem wenigstens einen zweiten Gasmelder als Bezugsnormal ausgebildet.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Dabei zeigen:
- FIG 1: ein System zur Feldkalibrierung von Gasmeldern (erstes Ausführungsbeispiel),
- FIG 2: ein System zur Feldkalibrierung von Gasmeldern (zweites Ausführungsbeispiel).

Sämtliche Figuren zeigen die Erfindung lediglich schematisch und mit ihren wesentlichen Bestandteilen. Gleiche Bezugszeichen entsprechen dabei Elementen gleicher oder vergleichbarer Funktion.

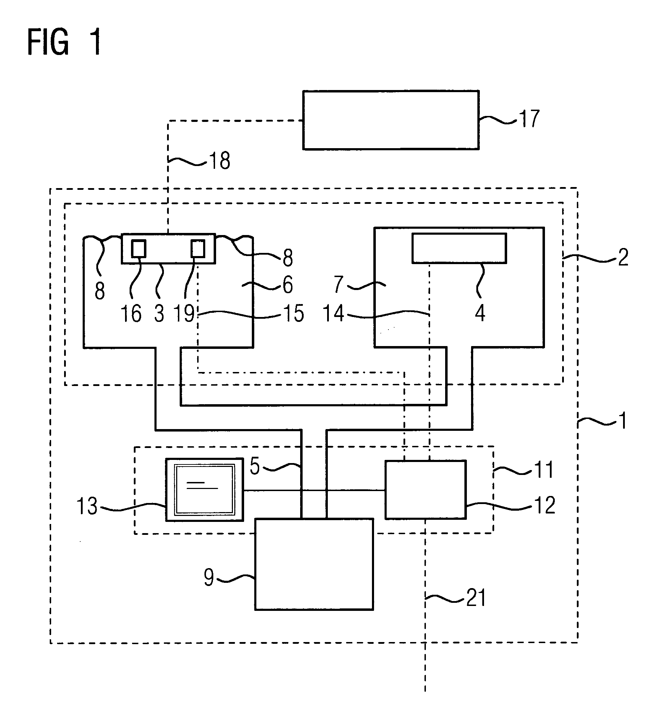

In einem ersten, in FIG 1 dargestellten Ausführungsbeispiel weist ein Prüfgerät 1 zur Feldkalibrierung einen Adapter 2 zur Aufnahme von zwei Gasmeldern 3, 4 auf. Sowohl ein zu kalibrierender, erster Gasmelder 3, als auch ein fabrikneuer, zweiter Gasmelder 4 werden einer identischen Referenzgaskonzentration ausgesetzt. Durch eine symmetrische Verteileranordnung 5 ist sichergestellt, dass jeweils die gleiche Menge an Referenzgas in die beiden identischen Prüfvolumen 6, 7 strömt.

Der das erste Prüfvolumen 6 bereitstellende Adapterteil wird über den ersten Gasmelder 3, der sich noch in seiner Einbauposition befindet, gestülpt und mit Hilfe von an dem Adaptergehäuse angebrachten, umlaufenden Dichtlippen 8 abgedichtet.

Das Adaptergehäuse ist in dem für den zweiten Gasmelder 4 vorgesehenen Prüfvolumen 7 derart ausgebildet, dass sich der mit dem Adapter verbundene zweite Gasmelder 4 in einer Position befindet, die hinsichtlich der Ein- und Durchströmverhältnisse des Referenzgases der Betriebsposition des ersten Gasmelders 3 nachgebildet ist oder einer solchen Position entspricht.

Zur Bereitstellung des Referenzgases umfasst das Prüfgerät 1 einen Gasgenerator 9, beispielsweise in Gestalt einer Druckflasche, mit dessen Hilfe eine unbekannte Menge an Referenzgas erzeugt und in das durch die beiden Teilvolumen 6, 7 gebildete Prüfvolumen eingespeist wird.

Das Prüfgerät 1 umfasst weiterhin eine Kalibrierelektronik 11 mit einer Auswerteeinheit 12 und einer Anzeigeeinheit 13. Die Anzeigeeinheit 13 ist ausgebildet zum Anzeigen der von dem zweiten Gasmelder 4 gemessenen Referenzkonzentration. Der zweite Gasmelder 4 ist mit der Kalibrierelektronik 11 über eine drahtgebundene oder drahtlose Kommunikationsleitung 14 verbunden.

Die Kalibrierung des zu prüfenden Gasmelders 3 wird in dem ersten Ausführungsbeispiel vorzugsweise wie folgt durchgeführt: Zur Überprüfung des sich in seiner Betriebsposition befindenden ersten Gasmelders 3 wird das Prüfgerät 1, an dessen Adapter 2 bereits der zweite, fabrikneue Gasmelder 4 befestigt ist, über den zu prüfenden Gasmelder 3 gestülpt, wobei Dichtlippen 8 aus Gummi den Adapter 2 abdichten und so im Inneren des Adapters 2 ein relativ gasdichter Raum entsteht. Eine absolute Gasdichtheit ist zu vermeiden, damit ein Ausströmen des Referenzgases möglich bleibt. In einem nächstem Schritt wird aus dem Gasgenerator 9 über den Verteiler 5 eine bestimmte, aber unbekannte Menge an Referenzgas in die beiden miteinander verbundenen, den Gasmeldern zugeordneten Teilvolumen 6, 7 eingelassen. Anschließend messen beide Gasmelder 3, 4 die Referenzgaskonzentration. Anhand der Unterschiede in den Messergebnissen wird der erste Gasmelder 3 kalibriert. Eine solche Kalibrierung ist dem Grunde nach dem Fachmann bekannt und soll daher nachfolgend auch nur beispielhaft beschrieben werden.

Die von dem zweiten Gasmelder 4 gemessene Gaskonzentration wird an die Kalibrierelektronik 11 übertragen und mit Hilfe der Anzeigeeinheit 13 der Kalibrierelektronik angezeigt.

Steht der erste Gasmelder 3 in einer direkten Kommunikationsverbindung mit dem Prüfgerät 1, beispielsweise in Gestalt einer drahtlosen optischen Kommunikationsverbindung 15, so überträgt der erste Gasmelder 3 die von ihm gemessene Gaskonzentration an die Kalibrierelektronik 11. Die Auswerteeinheit 12 der Kalibrierelektronik berechnet aus den Messergebnissen der beiden Gasmelder 3, 4 unmittelbar die Empfindlichkeit des ersten Gasmelders 3 und speichert das Ergebnis in einem hierfür vorgesehenen Speicherplatz 16 in dem ersten Gasmelder 3 ab. Dabei werden die dort zuvor hinterlegten Werte überschrieben.

Besteht keine Kommunikationsverbindung zwischen dem ersten Gasmelder 3 und dem Prüfgerät 1, sondern nur mit einem entfernten Empfänger 17, beispielsweise einer Brandmeldezentrale, so sendet der erste Gasmelder 3 über die bestehende Verbindung, beispielsweise ein Bussystem 18, das eine bidirektionale Kommunikation ermöglicht, die gemessene Gaskonzentration an den entfernten Empfänger 17. Der zu prüfende Gasmelder 3 wird in diesem Fall vor Beginn der Kalibrierung in einen Testmodus geschaltet, beispielsweise durch Betätigen eines Schalters 19. Die Kalibrierelektronik 11 ist für einen solchen Fall derart ausgebildet, dass die Auswerteeinheit 12 die gemessene Gaskonzentration des zweiten Gasmelders 4 ebenfalls an den entfernten Empfänger 17 sendet, zu welchem Zweck eine Kommunikationsverbindung 21 vorgesehen ist. Bei dem entfernten Empfänger 17 werden die empfangenen Informationen abgespeichert und aus dem Verhältnis der beiden gemessenen Konzentrationen wird eine Korrektur der Empfindlichkeit des ersten Gasmelders 3 berechnet und über die bestehende Kommunikationsverbindung 15 an den ersten Gasmelder 3 gesendet.

Somit kann entweder eine Korrektur der alterungsbedingten Messabweichungen direkt in dem ersten Gasmelder 3 erfolgen oder aber ein entfernter Empfänger 17 errechnet aus den erhaltenen Daten die Alterung und berücksichtigt diese bei der Bewertung der Messergebnisse, wobei letzteres zumeist bei Anlagen praktiziert wird, bei denen eine Korrektur direkt im Gasmelder 3 aus Sicherheitsgründen nicht zugelassen ist.

Über die zuvor beschriebene Möglichkeit der Kommunikation zwischen Gasmelder 3 und Zentrale 17 hinaus sind weitere Varianten möglich. Beispielsweise kann der Gasmelder 3 über das Prüfgerät 1 drahtgebunden oder drahtlos mit der Zentrale 17 kommunizieren oder der Gasmelder 3 kommuniziert über das Prüfgerät 1 mit einer Upload-Datenbank oder generell mit einem übergeordneten Managementsystem, zum Beispiel einem System mit einer Melderkalibrierdatenverwaltung, welches weiterhin mit der Zentrale 17 kommuniziert.

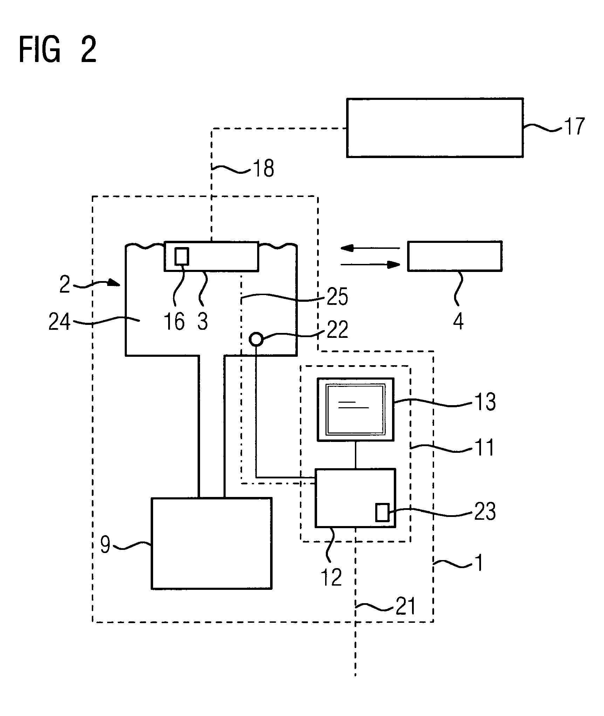

In dem in FIG 2 dargestellten zweiten Ausführungsbeispiel ist das Prüfgerät 1 weniger komplex aufgebaut. Insbesondere ist der Adapter 2 derart ausgeführt, dass er lediglich jeweils mit einem Gasmelder 3, 4 verbindbar ist.

Das Prüfgerät 1 verfügt in diesem Fall über einen oder mehrere Referenzgassensoren 22, welche die Konzentration des Referenzgases in dem Prüfvolumen 24 messen. Die Verwendung mehrerer Sensoren erhöht dabei die Zuverlässigkeit der Referenzmessung. Besonders vorteilhaft ist es, wenn durch einen modularen Aufbau eine einfache Austauschbarkeit der Referenzsensoren gewährleistet ist. Dies ist jedoch erst erforderlich, wenn sie so stark gealtert sind, dass sie nicht mehr zu ihrer ursprünglichen Empfindlichkeit gebracht werden können.

Bei diesem Ausführungsbeispiel verfügt das Prüfgerät 1 über zwei Betriebsmodi und die Kalibrierung erfolgt vorzugsweise wie folgt: Der Adapter 2 wird über den fabrikneuen Gasmelder 4 gestülpt und die Kalibrierelektronik 11 wird in den Modus "Kalibrierung des Referenzsensors" umgeschaltet, beispielsweise durch Betätigen eines an der Kalibrierelektronik 11 vorgesehenen Schalters 23. Danach wird eine unbekannte Menge von Referenzgas in das Innere des Adapaters 2 eingespeist. Der Referenzgassensor 22 des Prüfgerätes 1 misst die Gaskonzentration des Referenzgases und überträgt die Messergebnisse an die Kalibrierelektronik 11. Zugleich kommuniziert der zweite Gasmelder 4 über eine Kommunikationsverbindung 25 mit dem Prüfgerät 1 und sendet die von ihm gemessene Konzentration des Referenzgases an die Kalibrierelektronik 11. Die Auswerteeinheit 12 berechnet aus den empfangenen Messergebnissen automatisch die Korrektur des Referenzgassensors 22 und speichert diese in den Speicher des prüfgeräteeigenen Referenzgassensors 22 ab. Messwerte und Abweichungen können zusätzlich mit Hilfe der Anzeigeeinheit 13 der Kalibrierelektronik 11 angezeigt werden. Wird die Messung mit weiteren Referenzgeräten wiederholt, werden also mehrere fabrikneue Gasmelder 4 gemessen, kann die Genauigkeit der Kalibrierung erhöht werden. Im Anschluss daran ist der Referenzgassensor 22 des Prüfgerätes 1 kalibriert.

Um den zu überprüfenden Gasmelder 3 zu kalibrieren, wird der fabrikneue Gasmelder 4 von dem Adapter 2 des Prüfgerätes 1 getrennt und der zu überprüfende Gasmelder 3 mit dem Adapter 2 verbunden. Zudem wird die Kalibrierelektronik 11 in den Modus "Kalibrieren eines Gasmelders" geschaltet, beispielsweise wieder mit Hilfe des Schalters 23. Diese Situation ist in FIG 2 dargestellt. Anschließend wird Referenzgas in das Prüfvolumen 24 eingespeist.

Der zuvor frisch kalibrierte Referenzgassensor 22 im Prüfgerät 1 misst die Gaskonzentration und überträgt das Messergebnis an die mit dem Referenzgassensor 22 verbundene Kalibrierelektronik 11. Anschließend wird, wie in der oben beschriebenen Ausführungsform, die Prüfung bzw. die Kalibrierung des ersten Gasmelders 3 durchgeführt. Hierzu berechnet die Auswerteeinheit 12 aus den Messergebnissen der beiden Gasmelder 3, 4 die Empfindlichkeit des ersten Gasmelders 3 und speichert das Ergebnis in einem hierfür vorgesehenen Speicherplatz 16 des ersten Gasmelders 3 ab und/oder übermittelt einem entfernten Empfänger 17 die Messergebnisse über das Bussystem 18 oder eine andere Kommunikationsverbindung. Auch in dieser Ausführungsvariante können die oben beschriebenen Kommunikationsmöglichkeiten zwischen Gasmelder 3 und Prüfgerät 1 bzw. zwischen Gasmelder 3 und Zentrale 17 angewendet werden.

Von Vorteil ist, dass die Reihenfolge der Messungen nicht zwingend vorgeschrieben ist. In einer weiteren Ausführungsvariante der Erfindung werden die Messungen der Referenzgaskonzentrationen, welche den Kalibrierungen zugrunde liegen, in einer anderen Reihenfolge durchgeführt, als zuletzt beschrieben. Dabei erfolgt zunächst die Messung der Referenzgaskonzentration mit dem Prüfgerät 1 und dem zu prüfendem Gasmelder 3. Anschließend erfolgt die Messung der Referenzgaskonzentration mit dem Prüfgerät 1 und dem zweiten Gasmelder 4. Aus diesen Messergebnissen, die in der Kalibrierelektronik 11 gespeichert werden, wird die Korrektur für den ersten Gasmelder 3 berechnet und an diesen Gasmelder 3 drahtlos direkt oder über die Zentrale 17 übertragen. Die Kalibrierung des Referenzgassensors 22 des Prüfgerätes 1 kann mit anderen Worten auch im Anschluss an die Messung mit dem zu prüfenden Gasmelder 3 erfolgen. Für die Berechnung der Kalibrierung ist die Reihenfolge der Messungen unbeachtlich. Die Erfindung betrifft Prüfgeräte 1 zur Feldkalibrierung von Gasmeldern 3. Die Erfindung macht sich die grundlegende Idee zu Nutze, fabrikneue Gasmelder 4 als Normale bei der Feldkalibrierung zu verwenden. Dabei werden die fabrikneuen Gasmelder 4 entweder unmittelbar als Gebrauchsnormale eingesetzt. In diesem Fall entfällt die regelmäßige Kalibrierung durch ein Bezugsnormal, da die fabrikneuen Gasmelder 4 immer als hinreichend genau gelten. Alternativ hierzu werden die fabrikneuen Gasmelder 4 mittelbar, nämlich als hinreichend genau kalibrierte Bezugsnormale für die als Gebrauchsnormale dienenden, im Prüfgerät 1 eingebauten Referenzgassensoren 22 eingesetzt. In beiden Fällen werden die Messwerte der Gassensoren in den zu kalibrierenden Gasmeldern 3 auf ein hinreichend kalibriertes System zurückgeführt, nämlich entweder direkt auf wenigstens einen fabrikneuen Gasmelder 4 als Gebrauchsnormal oder aber auf einen mit Hilfe wenigstens eines solchen fabrikneuen Gasmelders 4 kalibrierten Referenzsensor 22 eines Prüfgerätes 1.

### Bezugszeichenliste

- 1: Prüfgerät
- 2: Adapter
- 3: erster Gasmelder
- 4: zweiter Gasmelder
- 5: Verteiler
- 6: Teilvolumen
- 7: Teilvolumen
- 8: Dichtlippe
- 9: Gasgenerator
- 10: (frei)
- 11: Kalibrierelektronik
- 12: Auswerteeinheit
- 13: Anzeigeeinheit
- 14: Kommunikationsverbindung
- 15: Kommunikationsverbindung
- 16: Speicherplatz
- 17: entfernter Empfänger, Zentrale
- 18: Kommunikationsverbindung, Bussystem
- 19: Schalter
- 20: (frei)
- 21: Kommunikationsverbindung
- 22: Referenzgassensor
- 23: Schalter
- 24: Prüfvolumen
- 25: Kommunikationsverbindung

## Patentansprüche

1. Prüfgerät zur Kalibrierung eines Gasmelders (3) im Feld, **dadurch gekennzeichnet,**
- **dass** das Prüfgerät (1) für eine gleichzeitige Messung der Gaskonzentration durch zwei Gasmelder (3, 4) ausgebildet ist,
- **dass** das Prüfgerät (1) einen Adapter (2) aufweist zum Herstellen einer Verbindung sowohl mit dem Gasmelder (3), als auch mit einem zweiten, insbesondere fabrikneuen Gasmelder (4) als Gebrauchsnormal, wobei sich die Empfindlichkeit des zweiten Gasmelders (4) seit seiner letzten Kalibrierung bei seiner Herstellung im Werk nur unwesentlich geändert hat, und
- **dass** das Prüfgerät (1) eine Kalibrierelektronik (11) aufweist, ausgebildet zur Kommunikation mit dem ersten und zweiten Gasmelder (3, 4), und zur Kalibrierung des Gasmelders (3) mit dem zweiten Gasmelder (4) nach einer gleichzeitigen Referenzmessung der Gaskonzentration eines Referenzgases durch beide Gasmelder (3, 4).

2. Prüfgerät nach Anspruch 1, **dadurch gekennzeichnet,**
**dass** die Kalibrierelektronik (11) ausgebildet zur Kommunikation mit einem entfernten Empfänger (17).

3. Prüfgerät nach Anspruch 2, **dadurch gekennzeichnet,**
**dass** die Kalibrierelektronik (11) eine Auswerteeinheit (12) zum Senden der gemessenen Gaskonzentration des zweiten Gasmelders (4) an den entfernten Empfänger (17) aufweist, falls keine Kommunikationsverbindung zwischen dem ersten Gasmelder (3) und dem Prüfgerät (1) besteht.

4. Prüfgerät nach einem der vorherigen Ansprüche,
**dadurch gekennzeichnet, dass** das Prüfgerät (1) ein gemeinsames Prüfvolumen zur Aufnahme von wenigstens zwei Gasmeldern (3, 4) aufweist.

5. Prüfgerät zur Kalibrierung eines ersten Gasmelders (3) im Feld, mit einem Adapter (2) für den ersten Gasmelder (3) oder für einen zweiten, insbesondere fabrikneuen Gasmelder (4), wobei der Adapter (2) derart ausgeführt ist, dass er jeweils mit nur einem Gasmelder (3, 4) verbindbar ist, wobei sich die Empfindlichkeit des zweiten Gasmelders (4) seit seiner letzten Kalibrierung bei seiner Herstellung im Werk nur unwesentlich geändert hat, und mit einem Referenzgassensor (22),
- wobei der Referenzgassensor (22) durch den zweiten Gasmelder (4) kalibrierbar ist und
- wobei das Prüfgerät (1) eine Schaltvorrichtung (11, 23) zum Umschalten des Betriebsmodus zwischen einem Modus zur Kalibrierung des Prüfgerätes (1) und einem Modus zur Kalibrierung des ersten Gasmelders (3) aufweist.

6. Prüfgerät nach Anspruch 5, **dadurch gekennzeichnet,**
- **dass** das Prüfgerät (1) eine Kalibrierelektronik (11) mit einer Auswerteeinheit (12) aufweist,
- **dass** die Kalibrierelektronik zur Kommunikation mit einem entfernten Empfänger (17) ausgebildet ist, und
- **dass** die Auswerteeinheit (12) ausgebildet ist zum Berechnen einer Empfindlichkeit des ersten Gassensors (3) aus den Messergebnissen der beiden Gasmelder (3, 4), und zum Übermitteln der Empfindlichkeit an den entfernten Empfänger (17).

7. Prüfgerät nach einem der vorherigen Ansprüche 2 bis 6, wobei der entfernte Empfänger (17) eine Brandmeldezentrale ist.

## Claims

1. Test device for calibrating a gas detector (3) in the field, **characterised in that**,
- the test device (1) is embodied for a simultaneous measurement of the gas concentration by means of two gas detectors (3, 4),
- the test device (1) has an adapter (2) for establishing a connection both with the gas detector (3) and also with a second, in particular brand new gas detector (4) as a measurement standard, wherein the sensitivity of the second gas detector (4) since its last calibration has only insignificantly changed during its manufacture on site, and
- the test device (1) has calibration electronics (11), embodied for communication with the first and second gas detector (3, 4) and for calibrating the gas detector (3) with the second gas detector (4) after a simultaneous reference measurement of the gas concentration of a reference gas by both gas detectors (3, 4).

2. Test device according to claim 1, **characterised in that** the calibration electronics (11) are embodied for communication with a remote receiver (17).

3. Test device according to claim 2, **characterised in that** the calibration electronics (11) have an evaluation unit (12) for sending the measured gas concentration of the second gas detector (4) to the remote receiver (17) if there is no communication link between the first gas detector (3) and the test device (1).

4. Test device according to one of the preceding claims, **characterised in that** the test device (1) has a common test volume for accommodating at least two gas detectors (3, 4).

5. Test device for calibrating a first gas detector (3) in the field, having an adapter (2) for the first gas detector (3) or for a second, in particular brand new gas detector (4), wherein the adapter (2) is embodied such that it can be connected in each case to just one gas detector (3, 4), wherein the sensitivity of the second gas detector (4) since its last calibration has only changed insignificantly during its manufacture on site and having a reference gas sensor (22),
- wherein the reference gas sensor (22) can be calibrated by the second gas detector (4) and
- wherein the test device (1) has a switching apparatus (11, 23) for switching the operating mode between a mode for calibrating the test device (1) and a mode for calibrating the first gas detector (3).

6. Test device according to claim 5, **characterised in that**,
- the test device (1) has calibration electronics (11) with an evaluation unit (12),
- the calibration electronics are embodied to communicate with a remote receiver (17), and
- the evaluation unit (12) is embodied to calculate a sensitivity of the first gas sensor (3) from the measurement results of the two gas detectors (3, 4) and to convey the sensitivity to the remote receiver (17).

7. Test device according to one of the preceding claims 2 to 6, wherein the remote receiver (17) is a fire alarm centre.

## Revendications

1. Appareil de contrôle pour le calibrage de champ d'un détecteur de gaz (3), **caractérisé en ce que**
- l'appareil de contrôle (1) est conçu pour une mesure simultanée de la concentration de gaz par deux détecteurs de gaz (3, 4),
- l'appareil de contrôle (1) présente un adaptateur (2) pour l'établissement d'un raccordement aussi bien avec le détecteur de gaz (3) qu'avec un deuxième détecteur de gaz (4), en particulier tout neuf, en usage normal, dans lequel la sensibilité du deuxième détecteur de gaz (4) n'a qu'insensiblement changé depuis son dernier calibrage lors de sa fabrication en usine, et
- l'appareil de contrôle (1) présente une électronique de calibrage (11), conçue pour la communication avec le premier et le deuxième détecteurs de gaz (3, 4), et pour le calibrage du détecteur de gaz (3) avec le deuxième détecteur de gaz (4) après une mesure de référence simultanée de la concentration de gaz d'un gaz de référence par les deux détecteurs de gaz (3, 4).

2. Appareil de contrôle selon la revendication 1, **caractérisé en ce que** l'électronique de calibrage (11) est conçue pour la communication avec un récepteur éloigné (17).

3. Appareil de contrôle selon la revendication 2, **caractérisé en ce que** l'électronique de calibrage (11) présente une unité d'évaluation (12) pour l'envoi de la concentration de gaz mesurée du deuxième détecteur de gaz (4) au récepteur éloigné (17), dans le cas où il n'existe aucune liaison de communication entre le premier détecteur de gaz (3) et l'appareil de contrôle (1).

4. Appareil de contrôle selon l'une quelconque des revendications précédentes, **caractérisé en ce que** l'appareil de contrôle (1) présente un volume de contrôle commun destiné à contenir au moins deux détecteurs de gaz (3, 4).

5. Appareil de contrôle pour le calibrage de champ d'un premier détecteur de gaz (3), avec un adaptateur (2) pour le premier détecteur de gaz (3) ou pour un deuxième détecteur de gaz (4), en particulier tout neuf, dans lequel l'adaptateur (2) est configuré de telle manière qu'il puisse n'être raccordé respectivement qu'à un détecteur de gaz (3, 4), dans lequel la sensibilité du deuxième détecteur de gaz (4) n'a qu'insensiblement changé depuis son dernier calibrage lors de sa fabrication en usine, et avec un détecteur de gaz de référence (22),
- dans lequel le détecteur de gaz de référence (22) peut être calibré par le deuxième détecteur de gaz (4), et
- dans lequel l'appareil de contrôle (1) présente un dispositif de commutation (11, 23) pour le basculement du mode de fonctionnement entre un mode de calibrage de l'appareil de contrôle (1) et un mode de calibrage du premier détecteur de gaz (3).

6. Appareil de contrôle selon la revendication 5, **caractérisé en ce que**
- l'appareil de contrôle (1) présente une électronique de calibrage (11) avec une unité d'évaluation (12),
- l'électronique de calibrage est conçue pour la communication avec un récepteur éloigné (17), et
- l'unité d'évaluation (12) est conçue pour le calcul d'une sensibilité du premier détecteur de gaz (3) à partir des résultats de mesure des deux détecteurs de gaz (3, 4), et pour la transmission de la sensibilité au récepteur éloigné (17).

7. Appareil de contrôle selon l'une quelconque des revendications précédentes 2 à 6, dans lequel le récepteur éloigné (17) est une centrale d'alarme incendie.
